# EUROPÄISCHE PATENTSCHRIFT

(11) **EP 1 312 302 B1**
(45) Veröffentlichungstag und Bekanntmachung des Hinweises auf die Patenterteilung: **09.01.2008**
(21) Anmeldenummer: 02020447.5
(22) Anmeldetag: 11.09.2002
(51) Int. Cl.: A61B 5/03

(54) **Vorrichtung zur intrakorporalen Messung des Hirndrucks**
Device for intracorporeal measurement of intracranial pressure
Dispositif pour mesurer la pression intracranienne à l'intérieur du corps

(30) Priorität: 16.11.2001 DE 10156469
(43) Veröffentlichungstag der Anmeldung: 21.05.2003
(73) Patentinhaber: Medos International S.a.r.l., 2400 Le Locle (CH)
(72) Erfinder: Dr. Bödecker, Volker, 30177 Hannover (DE); Dipl. Ing. Meyer, Stefan, 35236 Breidenbach (DE)
(74) Vertreter: Heselberger, Johannes

(56) Entgegenhaltungen:
- DE-A- 19 705 474
- DE-A- 19 713 266
- DE-A- 19 858 172
- DE-A- 19 945 879

## Beschreibung

Die Erfindung betrifft eine Vorrichtung zur intrakorporalen Messung des Hirndruckes nach dem Oberbegriff des Patentanspruches 1.

### [Stand der Technik]

Bei einer derartigen aus der DE 197 05 474 A1 bekannten Vorrichtung wird eine epidural implantierbare Messeinheit verwendet, bei welcher auf einer flexiblen Trägerfolie ein Sensorelement und eine Telemetrieeinrichtung mit einer induktiven Spule angeordnet sind. Das Sensorelement und die Telemetrieeinheit sind über Leiterbahnen elektrisch miteinander verbunden. Die implantierbare Messeinheit ist mit einer Silikonschicht zum Patientenschutz überzogen. Durch induktive Kopplung werden die vom Sensorelement erfassten Messdaten an eine extrakorporale Telemetrieeinrichtung übertragen und in einer Auswerteeinrichtung ausgewertet.

In der DE 199 45 879 wird eine Vorrichtung zum Messen von physikalischen Größen im Auge, insbesondere des Augeninnendrucks, beschrieben. Die Vorrichtung weist ein faltbares Telemetriesystem auf, welches eine auf einem faltbaren Träger flächig angeordnete Spule aufweist. Die Spule ist zusammen mit einem die Elektronik des Telemetriesystems enthaltenden Baustein vollständig im biokompatiblen Implantatmaterial eingegossen.

Die DE 197 13 266 offenbart ein Verfahren zur Verkapselung eines Meßsystems welches zu medizinischen Zwecken, vor allem aber zur Hirndruckmessung, dient. Das Meßsystem wird verkapselt, indem ein unterer Streifen auf eine untere Werkzeughälfte aufgelegt und in die Stege der unteren Werkzeughälften eingewalzt wird. Dann wird eine Taperfolie auf den unteren Streifen und ein oberer Streifen auf die Taperfolie aufgelegt. Anschließend wird die obere Werkzeughälfte auf den oberen Streifen gelegt und die Werkzeughälften werden zusammengepresst. Die Streifen werden dann ausvulkanisiert und danach getempert.

Aus der DE 198 58 172 ist ein Implantat zur Messung des Augeninnendrucks bekannt. Das Implantat umfasst eine künstliche Linse, einen an den Rand der Linse angeformten Randwulst und nach außen abstehende Haltearme an dem Randwulst sowie ein telemetrisches Endosystem. Das telemetrische Endosystem weist einen Drucksensor, eine Signalverarbeitungsschaltung und Telemetrieeinrichtungen für die drahtlose Übertragung der Sensorsignale und den Empfang der Speiseenergie auf und ist auf dem Randwulst um die künstliche Linse herum angeordnet.

Schließlich beschreibt die DE 197 05 474 eine implantierbare Meßeinheit zur intrakorporalen Messung von Patientendaten, insbesondere von Hirndrücken. Die Meßeinheit ist zum mobilen Einsatz unter Alltagsbedingungen geeignet und umfasst mindestens ein intrakorporal einsetzbares Sensorelement und eine damit verbundene Telemetrieeinheit zur induktiven Energieübertragung und Datenübermittlung. Dabei sind das mindestens eine Sensorelement und die Telemetrieeinheit auf einer flexiblen Folie aufgebracht und die flexible Folie umfasst Leiterbahnen zur elektrischen Verbindung des mindestens einen Sensorelements und der Telemetrieeinheit.

### [Aufgabe der Erfindung]

Aufgabe der Erfindung ist es, eine eingangs genannte Vorrichtung zu schaffen, welche bei chipseitig kleiner Druckaufnehmerfläche eine sichere Messung von Hirndrücken bei Lebewesen gewährleistet.

Diese Aufgabe wird erfindungsgemäß durch die kennzeichnenden Merkmale des Patentanspruches 1 gelöst.

In den Unteransprüchen sind vorteilhafte Weiterbildungen der Erfindung dargestellt.

Hierzu wird eine Messeinrichtung verwendet, welche vorzugsweise epidural implantiert wird. Diese Messeinrichtung beinhaltet einen Druckaufnehmer und die Telemetrieelektronik, welche in einem Chip angeordnet sind. Die Telemetrieelektronik ist mit der induktiven Spule durch Bonden direkt elektrisch verbunden. Der Chip und die induktive Spule sind mit überlappender Anordnung auf einer flexiblen Trägerfolie vorgesehen oder in einem flexiblen Träger oder einer flexiblen Umhüllung eingebettet. Zumindest der Chip und der vom Chip überlappte Spulenteil sind von einem Druck übertragenden Medium, welches in einer flexiblen Umhüllung eingeschlossen ist, eingebettet. Zumindest die flexible Umhüllung besteht vorzugsweise aus biokompatiblen Material. Auch das Druck übertragende Medium kann aus einem biokompatiblen Werkstoff bestehen. Die Umhüllung wird von einer flexiblen spannungsfreien Folie aus einem biokompatiblen Werkstoff gebildet. Das Druck übertragende, biokompatible Medium wird von einem Gas, von einer Flüssigkeit, von einem Gel oder einem Öl, insbesondere auf Silikonbasis gebildet.

Der Chip zeichnet sich durch ein geringes Volumen aus und kann in der Höhe beispielsweise kleiner als 1 mm, insbesondere kleiner als 0,5 mm bemessen sein. Die Chipfläche kann wenige mm² betragen. Die Spule kann ebenfalls mit geringer Höhe, z.B. ca. 20 µm als Planarspule oder mit ebenfalls geringer Höhenabmessung als vorzugsweise verformbare gewickelte Spule ausgebildet werden. Die Spule kann mit mikrosystemtechnischen Verfahren, z.B. mikrogalvanisch, insbesondere photolithographisch hergestellt werden. Die Gesamthöhe der Messeinheit, einschließlich der Trägerfolie kann kleiner als 1 mm bemessen werden.

Bei der Erfindung kann mit zwei Komponenten, nämlich dem Chip und der induktiven Spule die Messwerterfassung, die Energieübertragung und die telemetrische Datenübertragung, insbesondere in digitaler Form durch geführt werden. Im Chip sind vorzugsweise der Druckaufnehmer, gegebenenfalls eine Temperaturmesseinrichtung, der Analog/Digital-Wandler und die Telemetrie-/Transponderelektronik untergebracht. Hierdurch erreicht man einen voll integrierten Chip, der neben dem telemetrischen Drucksensor, gegebenenfalls mit einem zusätzlichen telemetrischen Temperatursensor ausgestattet ist. Die telemetrisch übertragenen Daten liegen vorzugsweise in digitaler Form vor. Die Anordnung des Chips und der Spule sowie die Verbindung des Chips mit der Spule durch Bonden können von der Art sein, wie sie aus der DE 199 45 879 A1 bekannt ist. Die Umhüllung und das Druck übertragende Medium besitzen eine ausreichende Wärmeleitfähigkeit, um die Temperaturmessung durchzuführen. Die Druckmessung wird vorzugsweise kapazitiv durchgeführt. Hierzu kann die druckempfindliche Oberfläche von einer oder mehreren Membranen gebildet werden, welche jeweils eine Kondensatorbelegung bei der kapazitiven Druckmessung bildet. Es können auch mehrere druckempfindliche Oberflächen, insbesondere Membranen vorgesehen sein.

Man erreicht mit einer derartigen Vorrichtung eine hohe Transponderreichweite von mehreren cm. Bei einer Transponderfrequenz zwischen 10 und 15 MHz, beispielsweise 13,56 MHz wird eine Reichweite von etwa 5 cm erreicht.

Bei der Erfindung wird eine flexible Anpassung des Sensorelementes an die unebenen Oberflächen am Implantationsort erreicht. Durch die Erfindung wird eine dreidimensional flexible Spule geschaffen mit Außenabmessungen zwischen 5 mm und 18 mm. Die Spule kann eine kreisringförmige Ausgestaltung oder eine elliptische Form aufweisen. Die Spule kann zusammen mit dem Chip in dem Druck übertragenden Medium innerhalb der Umhüllung eingebettet sein. Falls nur ein Teil der Spule zusammen mit dem Chip im Druck übertragenden Medium eingebettet ist, ist es von Vorteil, die induktive Spule im flexiblen Träger einzubetten. Bei der Erfindung wird eine implantierbare Messeinrichtung geschaffen, welche sich in der Geometrie den anatomischen Gegebenheiten am Implantationsort anpasst. Bei der implantierbaren Messeinrichtung gemäß der Erfindung kann ein einzelner Chip mit geringen Abmessungen und somit kleiner Druckaufnahmefläche verwendet werden. Aufgrund des Druck übertragenden Mediums, in welches der Chip eingebettet ist, liegt am Messort bzw. Implantationsort durch die vergrößerte Außenfläche der Messeinrichtung eine bedeutend größere Druckaufnahmefläche vor, auf welche der zu messende Hirndruck wirkt. Vorzugsweise sind am Chip für die Druckmessung mehrere beispielsweise vier Druckaufnahmeflächen, insbesondere Membranen für die kapazitive Druckmessung vorgesehen.

Am Chip können ferner insbesondere passivierte Druckmessstellen vorgesehen sein, welche mit einen fest vorgegebenen (standardisierten) Druck beaufschlagt sind. Für die Erfassung des Hirndruckes wird die Differenz zwischen dem vom Druck übertragenden Medium vermittelten Druck und dem standardisierten Druck verwendet. Hierdurch erreicht man eine Kompensation von Driften und anderer Einflüsse auf die Druckaufnehmer. Da keine elektrischen Verbindungsleitungen zwischen der Spule und dem Chip vorgesehen sind, sondern der Chip direkt beispielsweise durch Flip-Chip-Bonden mit der dreidimensional flexiblen Spule verbunden ist, können geringe, an den Mess- bzw. Implantationsort angepasste Außenabmessungen der implantierbaren Messeinrichtung erreicht werden. Aufgrund der Ein-Chip-Lösung ist die Störanfälligkeit der Messeinrichtung im Vergleich zu Systemen mit mehreren Modulen gering.

Die Messeinrichtung wird nach ihrer Umhüllung mit dem Druck übertragenden Medium in der Hülle, welche flexibel und reißfest ist und zum Verschließen beispielsweise verklebt, vulkanisiert oder verschweißt werden kann, kalibriert. Ferner ist die Hülle sterilisierbar.

Jeder Chip kann eine spezifische Kennung, die bei jeder telemetrischen Messung abgefragt und von der die Messdaten verarbeitenden Auswerteeinrichtung zugeordnet wird, erhalten. Auf diese Weise können mehrere implantierbare Messeinrichtungen beispielsweise bis zu mehrere Hundert Messeinrichtungen mit einer Auswerteeinrichtung bearbeitet werden. Die Abfragung kann mit einem telemetrischen Abfrage-/Lesegerät durchgeführt werden. Bei der Kalibrierung der Messeinrichtung wird eine bestimmte Kennlinie ermittelt. Diese Kennlinie wird vorzugsweise im Abfrage-/Lesegerät und/oder in der Auswerteeinrichtung zugeordnet zu der spezifischen Kennung des Chips gespeichert. Hierdurch erkennt das Abfrage-/Lesegerät und/oder die Auswerteeinrichtung bei der telemetrischen Messung die jeweilige Messeinrichtung und berücksichtigt bei der Auswertung die hierzu gehörige Kennlinie.

Das Abfrage-/Lesegerät und die Auswerteeinrichtung und ein damit verbundenes Aufzeichnungsgerät können in einer Baueinheit, welches vorzugsweise als Handgerät ausgebildet ist, untergebracht sein. In diesem Gerät ist auch die extrakorporale Induktionsspule, welche an die extrakorporale Telemetrieeinrichtung angeschlossen ist, integriert. Das Abfrage-/Lesegerät kann über ein Kabel mit der Auswerteeinrichtung verbunden sein. Es ist jedoch auch möglich, durch Funkübertragung die Messdaten vom Abfrage-/Lesegerät auf die Auswerteeinrichtung zu übertragen.

### [Beispiele]

Anhand der Figuren wird die Erfindung noch näher erläutert. Es zeigt
- Fig. 1: eine Draufsicht auf eine implantierbare Messeinrichtung, welche ein Ausführungsbeispiel der Erfindung ist;
- Fig. 2: in teilweise geschnittener Darstellung entlang einer Schnittlinie A-A das Ausführungsbeispiel der Fig. 1;
- Fig. 3: eine schematische Seitenansicht einer weiteren Ausführungsform einer implantierbaren Messeinrichtung;
- Fig. 4: eine epidural implantierte Messeinrichtung mit einem von außen in die Nähe der implantierten Messeinrichtung gebrachten telemetrischen Abfrage-/Lesegerät;
- Fig. 5: ein Ausführungsbeispiel für das telemetrische Abfrage-/Lesegerät; und
- Fig. 6: ein Blockschaltbild für die Zuordnung unterschiedlicher Messdaten zu den zugehörigen Messeinrichtungen.

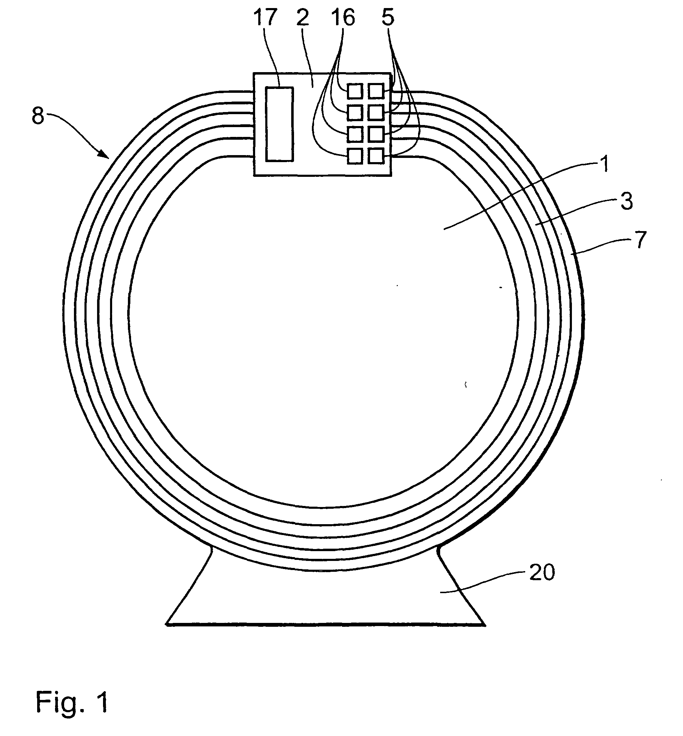

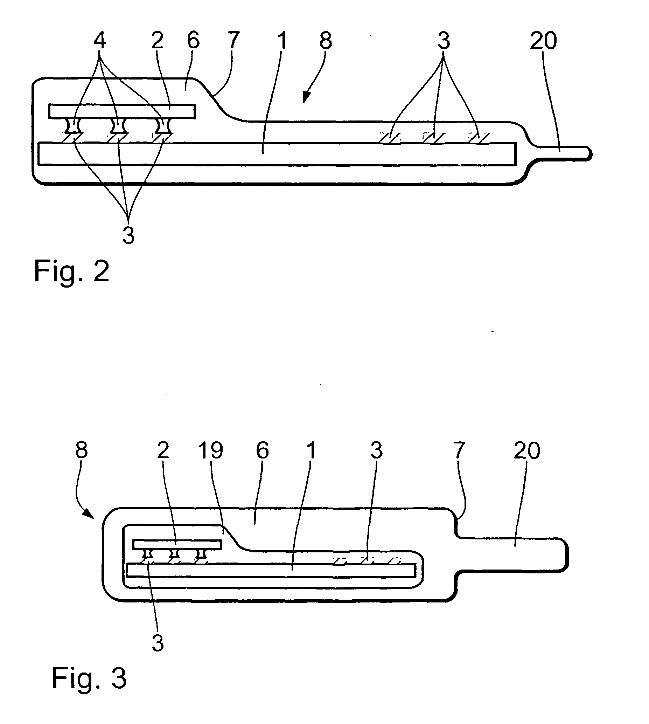

Die in den Fig. 1 und 2 dargestellte implantierbare Messeinrichtung beinhaltet eine flexible Trägerfolie 1, an welcher ein einziger hybrid aufgebauter Chip 2 und eine induktive Spule 3 mit überlappender Anordnung vorgesehen sind. Der Chip 1 beinhaltet einen Druckaufnehmer (Drucksensor) mit einer oder mehreren druckaufnehmenden Flächen 5. Beim dargestellten Ausführungsbeispiel sind vier Druck aufnehmende Flächen 5, welche als Membranen ausgebildet sein können, dargestellt. Die Membranen sind Bestandteil, insbesondere Kondensatorbelegungen eines kapazitiv messenden Drucksensors. Ferner können zusätzliche Drucksensoren 16 vorgesehen sein, welche einen fest vorgegebenen Druck (Standarddruck) messen. Die Messsignale, welche von der Messeinrichtung gebildet werden, ergeben sich aus der Differenz der Druckwerte, welche an den Druck aufnehmenden Flächen 5 und den Standarddrucksensoren 16 gemessen werden. Die Standartdrucksensoren 16 sind als passivierter Sensoren ausgebildet und besitzen im Aufbau zu den Druck aufnehmenden Flächen 5 identische Zellen. Die Druck aufnehmenden Flächen 5 messen den zu erfassenden Hirndruck. Durch Bildung der Druckdifferenz werden Driften der Drucksensoren kompensiert. Der Chip 2 beinhaltet ferner einen Temperaturfühler 17, welcher der am. Messort bzw. Implantationsort herrschenden Temperatur proportionale Signale erzeugt. Ferner sind im Chip 2 ein Analog-/Digitalwandler sowie eine Telemetrie-/Transponderelektronik vorgesehen. Die Elektronik ist über eine elektrische Kontaktierung 4, insbesondere Flip-Chip-Bonden direkt mit der induktiven Spule 3 kontaktiert.

Beim dargestellten Ausführungsbeispiel ist die induktive Spule 3 in der vorzugsweise flexibel ausgebildeten Trägerfolie 1 angeordnet und durch eine Schutzfolie 18 abgedeckt. Die induktive Spule 3 ist auf einer der beiden Oberfläche der Trägerfolie 1 angeordnet und der Chip 2 ist auf der gleichen Oberfläche angeordnet. Er kann auch aus der gegenüberliegenden Oberfläche der Trägerfolie 1 vorgesehen sein. Wie insbesondere aus der Fig. 1 zu ersehen ist, sind der Chip 2 und ein dem Chip 2 benachbarter Spulenbereich von einer Umhüllung 7, welche als flexible Hüllfolie ausgebildet ist, umgeben. Innerhalb der Umhüllung 7 befindet sich ein Druck übertragendes Medium, insbesondere eine biokompatible Flüssigkeit, beispielsweise Öl auf Silikonbasis. Der Chip 2 und der benachbarte Spulenbereich werden druckdicht durch das Druck übertragende Medium 6 umhüllt bzw. sind im Druck übertragenden Medium 6 eingebettet. Die Umhüllung 7 kann mit der Trägerfolie 1 verschweißt, verklebt oder durch Vulkanisieren fest mit der Trägerfolie 1 verbunden sein.

Die Umhüllung 7 kann zusätzlich auch die gesamte Spule 3 umhüllen. Gegebenenfalls kann das Druck übertragende Medium 6 dann auch im Bereich der umhüllten Spule 3 vorgesehen sein. Auch bei dieser Ausführungsform kann die Umhüllung 7 durch Verschweißen, Verkleben, Vulkanisieren druckdicht verschlossen sein. Wie aus der Fig. 3 zu ersehen ist, kann die aus dem einzigen Chip 2 und der Spule 3 bestehende und auf der Trägerfolie 1 vorgesehene Anordnung mit einer Schutzschicht 19, insbesondere gummielastischen Schutzschicht, beispielsweise aus Silikonkautschuk umhüllt sein. Diese Anordnung ist eingebettet in das von der Umhüllung 7 umschlossene Druck übertragende Medium 6.

Die Trägerfolie 1 kann vorzugsweise aus einem Polyimid gebildet sein. Für die Umhüllung 7 eignet sich beispielsweise eine Polyurethanfolie. Für das Druck übertragende Medium 6 eignen sich Silikonöl und/oder semifluorierte Alkane.

Ferner kann an die Umhüllung 7 eine Lasche 20 angeformt sein, mit welcher eine genaue Positionierung der Messeinrichtung 8 am Implantationsort und ein Entfernen der Messeinrichtung vom Implantationsort mit Hilfe eines geeigneten chirurgischen Greifwerkzeugs erleichtert wird.

Die Umhüllung 7 umgibt das Druck übertragende Medium 6 vorzugsweise spannungsfrei, so dass eine ungestörte Übertragung des zu messenden Hirndruckes auf die druckempfindliche Sensorfläche 5 des im Chip 2 integrierten Druckaufnehmers erreicht wird. Die Kalibrierung der Messeinrichtung 8 erfolgt vorzugsweise nachdem die Umhüllung 7 geschlossen ist, d.h. nach Fertigstellung der implantierbaren Messeinrichtung 8.

Die Windungen der Spule 3 sind vorzugsweise mit mikrosystemtechnischen, insbesondere photolithographischen Methoden hergestellt. Die Spulenwindungen können als planare Spulenwindungen nebeneinander und/oder übereinander liegend in oder auf der Trägerfolie 1 angeordnet sein. Es ist auch möglich, eine aus einem dünnen Leiter hergestellte verformbare gewickelte Spule zu verwenden. Die Spule kann beispielsweise eine Höhe von 20 µm aufweisen. Ferner sind Trägerfolien, die bis zu einer Dicke von 8 µm reduziert sein können, herstellbar. Beim Einbetten der Spule in den flexiblen Träger können zwei oder mehr derartige Trägerfolien zum Einbetten verwendet werden. Auf diese Weise lässt sich eine Messeinrichtung 8 mit einer Gesamthöhe bzw. Gesamtdicke von geringer als 2 mm herstellen.

Die Messeinrichtung 8 wird zur Hirndruckmessung nach Eröffnen der Kopfhaut 13 und des Schädeldaches 12 eines Patienten implantiert. Der Implantationsort kann subdural oder epidural liegen. Vorzugsweise wird die Messeinrichtung 8 epidural implantiert, wie aus der Fig. 3 zu ersehen ist. Nach der Implantation befindet sich somit die Messeinrichtung 8 zwischen dem Schädeldach 12 und der harten Hirnhaut 11 (Dura mater encephali).

Zur Durchführung der Messung wird extrakorporal ein Abfrage-/Lesegerät 10 in die Nähe der implantierten Messeinrichtung 8 gebracht. Das Abfrage-/Lesegerät 10 besitzt eine induktive Spule 9 zur telemetrischen Energieübertragung auf die induktive Spule 3 der Messeinrichtung und zum Empfang der telemetrisch übertragenen Messdaten der implantierten Messeinrichtung 8. Zumindest die induktive Spule 9 des Abfrage-/Lesegerätes 10 kann am Kopf des Patienten in Transponderreichweite befestigt sein. Hierdurch können Messdaten ständig abgefragt werden. Das Abfrage-/Lesegerät 10 ist vorzugsweise als Handgerät ausgebildet und kann über ein Kabel 15 an eine Auswerteeinrichtung 21 mit Anzeigeeinrichtung 14 angeschlossen sein. Es ist jedoch auch möglich, die Auswerteeinrichtung und eine Anzeigeeinrichtung 14 im Gerät 10 zu integrieren. Ferner kann das Abfrage-/Lesegerät 10 über Funk die Messdaten an die Auswerteeinrichtung 21 weitergeben.

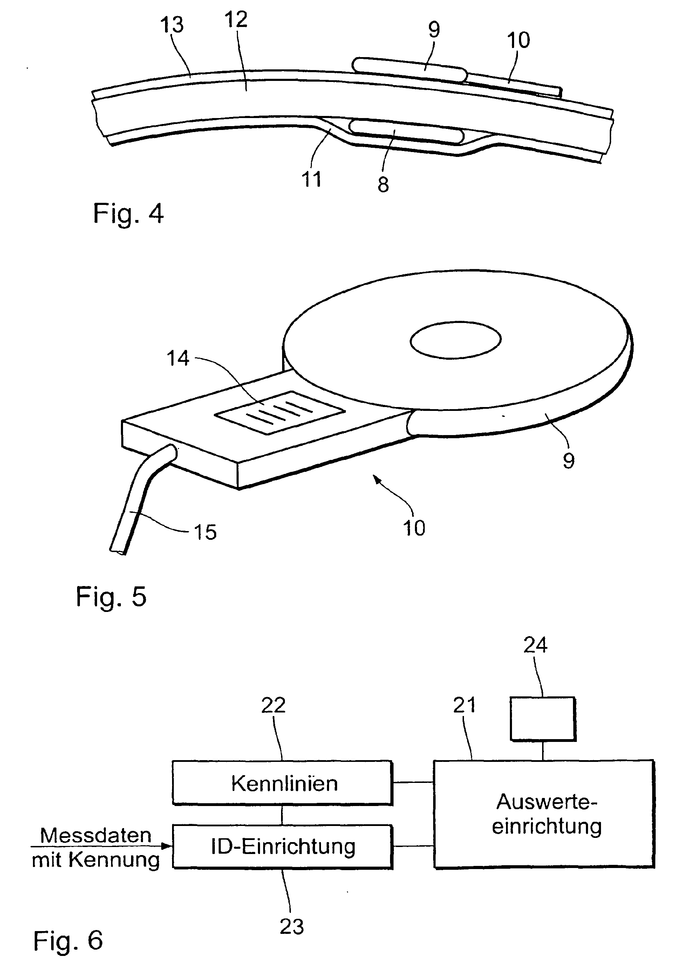

Aus der Fig. 6 ist zu ersehen, dass die Auswerteeinrichtung 21 an einen Speicher 22 angeschlossen ist oder diesen enthält, in welchem Kennlinien, die mehreren implantierten Messeinrichtungen zugeordnet sind, gespeichert sind. Ferner ist die Auswerteeinrichtung an eine Kennungs- bzw. Identifizierungseinrichtung 23 angeschlossen, in welchem Identifizierungscodes bzw. spezifische Kennungen für die jeweiligen Messeinrichtungen 8 erkannt werden. Auch die Identifizierungseinrichtung 23 kann Bestandteil der Auswerteeinrichtung 21 sein. Die im Speicher 22 enthaltenen Kennlinien sind den jeweiligen Identifizierungscodes bzw. spezifischen Kennungen der Messeinrichtung zugeordnet. Die vom Abfrage-/Lesegerät 10 kommenden Messdaten enthalten jeweils die spezifische Kennung der Messeinrichtung, zu welcher die Messdaten gehören. Diese spezifische Kennung wird in der Kennungseinrichtung 23 für die Auswertung erkannt. Die Auswerteeinrichtung 21 ruft für die Auswertung der Messdaten die im Speicher 22 gespeicherte Kennlinie ab. Die Auswerteeinrichtung kann mit einem Sensor 24 verbunden sein, mit dem der Umgebungsluftdruck gemessen wird. Der Umgebungsluftdruck bildet den Referenzwert zu dem ermittelten Hirndruck.

### [Bezugszeichenliste]

- 1: Trägerfolie
- 2: Chip
- 3: induktive Spule
- 4: elektrische Kontaktierung (Flip-Chip-Bonden)
- 5: Drucksensorfläche
- 6: Druck übertragendes Medium
- 7: Umhüllung (flexible Hüllfolie)
- 8: implantierbare Messeinrichtung
- 9: externe induktive Spule
- 10: Abfrage-/Lesegerät
- 11: harte Hirnhaut (Dura mater encephali)
- 12: Schädeldach
- 13: Kopfhaut
- 14: Anzeigeeinrichtung
- 15: Kabel
- 16: Standarddrucksensor
- 17: Temperaturfühler
- 18: Schutzfolie
- 19: Schutzschicht
- 20: Lasche
- 21: Auswerteeinrichtung
- 22: Kennlinienspeicher
- 23: Identifizierungseinrichtung
- 24: Sensor für Umgebungsluftdruck

## Patentansprüche

1. Vorrichtung zur intrakorporalen Druckmessung, insbesondere Hirndruckmessung mit einer implantierbaren Messeinrichtung (8), die auf oder in einem Träger ein Sensorelement und eine Telemetrieeinrichtung mit einer daran angeschlossenen induktiven Spule (3) aufweist, welche in einer flexiblen Umhüllung (7) angeordnet sind, und einem extrakorporalen an eine Auswerteeinrichtung (21) anschliessbaren telemetrischen Abfrage-/Lesegerät (10), wobei der Druckaufnehmer und die Telemetrieelektronik, in einem direkt mit der induktiven Spule (3) kontaktierten Chip (2) angeordnet sind, wobei zumindest der Chip (1) und der vom Chip (1) überlappte Spulenteil in einem Druck übertragenden Medium (6), welches von der flexiblen Umhüllung (7) umschlossen ist, eingebettet sind,
**dadurch gekennzeichnet, dass**
das Druck übertragende Medium (6) eine Flüssigkeit, Gas, Gel oder Öl, insbesondere Öl auf Silikonbasis ist und dass die flexible Umhüllung (7) aus einer spannungsfreien Folie besteht.

2. Vorrichtung nach Anspruch 1, **dadurch gekennzeichnet, dass** die implantierbare Messeinrichtung (8) mit fertiggestellter Umhüllung (7) kalibriert ist.

3. Vorrichtung nach einem der Ansprüche 1 bis 2, **dadurch gekennzeichnet, dass** der Chip (1) ferner einen Temperaturfühler (17) aufweist.

4. Vorrichtung nach einem der Ansprüche 1 bis 3, **dadurch gekennzeichnet, dass** die induktive Spule (3) als verformbare Planarspule oder verformbare gewickelte Spule ausgebildet ist.

5. Vorrichtung nach einem der Ansprüche 1 bis 4, **dadurch gekennzeichnet, dass** die induktive Spule (3) auf oder in einem flexiblen Träger eingebettet ist.

6. Vorrichtung nach einem der Ansprüche 1 bis 5, **dadurch gekennzeichnet, dass** die Aussenabmessungen der induktiven Spule (3) weniger als 18 mm, insbesondere etwa 12 mm betragen.

7. Vorrichtung nach einem der Ansprüche 1 bis 6, **dadurch gekennzeichnet, dass** die Dicke der implantierbaren Messeinrichtung etwa 2 mm oder weniger beträgt.

8. Vorrichtung nach einem der Ansprüche 1 bis 7, **dadurch gekennzeichnet, dass** die induktive Spule (3) dreidimensional flexibel ausgebildet ist.

9. Vorrichtung nach einem der Ansprüche 1 bis 8, **dadurch gekennzeichnet, dass** eine extrakorporale induktive Spule (9), welche mit einem Abfrage-/Lesegerät (10) verbindbar ist, am Kopf des Patienten in der Nähe der implantierten Messeinrichtung (8) befestigbar ist.

10. Vorrichtung nach einem der Ansprüche 1 bis 9, **dadurch gekennzeichnet, dass** die extrakorporale Telemetrieeinrichtung als Handgerät ausgebildet ist, in welchem das Abfrage-/Lesegerät (10) vorgesehen ist.

11. Vorrichtung nach Anspruch 10, **dadurch gekennzeichnet, dass** das Handgerät eine Anzeigeeinrichtung (14) aufweist.

12. Vorrichtung nach einem der Ansprüche 1 bis 11, **dadurch gekennzeichnet, dass** die Auswerteeinrichtung (21) die Messdaten mit Hilfe einer der jeweiligen Messeinrichtung (8) zugeordneten Kennlinie auswertet.

13. Vorrichtung nach einem der Ansprüche 1 bis 12, **dadurch gekennzeichnet, dass** die Messdaten mehrerer implantierbarer Messeinrichtungen (8) von einer Auswerteeinrichtung (21) auswertbar sind.

14. Vorrichtung nach Anspruch 13, **dadurch gekennzeichnet, dass** die Messdaten den jeweiligen Messeinrichtungen (8) zugeordnete spezifische Kennungen aufweisen, mit denen den jeweiligen Messeinrichtungen (8) zugeordneten Kennlinien aus einen Kennlinienspeicher (23) bei der Auswertung abrufbar sind.

15. Vorrichtung nach einem der Ansprüche 1 bis 14, **dadurch gekennzeichnet, dass** die Messdaten die Druckdifferenz zwischen dem vom Drucksensor (5) gemessenen Hirndruck und einem von einem Standarddrucksensor (16) gemessenen vorgegebenen Druck beinhalten.

16. Vorrichtung nach einem der Ansprüche 1 bis 15, **dadurch gekennzeichnet, dass** die Auswerteeinrichtung (21) mit einem Sensor (24) zur Erfassung des Umgebungsluftdruckes verbunden ist.

## Claims

1. Device for intracorporeal pressure measurement, especially measurement of the intracranial pressure, by means of an implantable measuring equipment (8), which comprises on or in a carrier a sensor element and a telemetry equipment with an inductive coil (3) connected thereto, which are arranged in a flexible covering (7) and an extracorporeal telemetric request-/reading device (10), being connectable to an analysis equipment (21), wherein the pressure transducer and the telemetry electronics are arranged in a chip (2) being directly contacted with the inductive coil (3), wherein at least the chip (1) and the coil portion overlapped by the chip (1) are embedded in a pressure exchanging medium (6), which is enclosed by the flexible covering (7)
**characterized in that**
the pressure exchanging medium (6) is a liquid, gas, gel or oil, especially oil based on silicon and **in that** the flexible covering (7) consists of a tension-free foil.

2. Device according to claim 1, **characterized in that** the implantable measuring equipment (8) is calibrated with the completed covering (7).

3. Device according to one of the claims 1 to 2, **characterized in that** the chip (1) further comprises a temperature sensor (17).

4. Device according to one of the claims 1 to 3, **characterized in that** the inductive coil (3) is formed as deformable planar coil or deformable winded coil.

5. Device according to one of the claims 1 to 4, **characterized in that** the inductive coil (3) is embedded on or in a flexible carrier.

6. Device according to one of the claims 1 to 5, **characterized in that** the outer dimensions of the inductive coil (3) are less than 18 mm, especially approx. 12 mm.

7. Device according to one of the claims 1 to 6, **characterized in that** the thickness of the implantable measuring equipment is approx. 2 mm or less.

8. Device according to one of the claims 1 to 7, **characterized in that** the inductive coil (3) is formed three-dimensionally flexible.

9. Device according to one of the claims 1 to 8, **characterized in that** the extracorporeal inductive coil (9), being connectible with a request-/reading device (10) is attachable at the head of the patient near the implanted measuring equipment (8).

10. Device according to one of the claims 1 to 9, **characterized in that** the extracorporeal telemetry equipment is formed as hand apparatus, in which the request-/reading device (10) is provided.

11. Device according to claim 10, **characterized in that** the hand apparatus comprises a display equipment (14).

12. Device according to one of the claims 1 to 11, **characterized in that** the analysis equipment (21) analysis the measuring data with the aid of a characteristic curve being assigned to the respective measuring equipment (8).

13. Device according to one of the claims 1 to 12, **characterized in that** the measuring data of several implantable measuring equipments (8) are to be analyzed by an analysis equipment (21).

14. Device according to claim 13, **characterized in that** the measuring data of the respective measuring equipment (8) comprises assigned specific identification, by means of which the characteristic curves being assigned to respective measuring equipments (8) can be requested from a characteristic curve storage (23) during analysis.

15. Device according to one of the claims 1 to 14, **characterized in that** the measuring data includes the pressure difference between the intracranial pressure measured by the pressure sensor (5) and a predetermined pressure measured by a standard pressure sensor (16).

16. Device according to one of the claims 1 to 15, **characterized in that** the analysis equipment (21) is connected with a sensor (24) for the recordation of the ambient air pressure.

## Revendications

1. Dispositif de mesure de la pression intracorporelle, en particulier de mesure de la pression intracrânienne, comprenant un dispositif de mesure implantable (8) qui comporte, sur ou dans un support, un élément capteur et un dispositif télémétrique auquel est connectée une bobine inductive (3) disposée dans une enveloppe souple (7), et comprenant un appareil télémétrique extracorporel d'interrogation/lecture (10) raccordable à un dispositif d'analyse (21), le capteur de pression et l'électronique de télémétrie étant disposés dans une puce (2) en contact direct avec la bobine inductive (3), au moins la puce (1) et la partie de bobine recouverte par la puce (1) étant incluses dans un milieu transmetteur de pression (6) qui est entouré par l'enveloppe souple (7), **caractérisé en ce que** le milieu transmetteur de pression (6) est un liquide, un gaz, un gel ou une huile, en particulier une huile à base de silicone, et **en ce que** l'enveloppe souple (7) est constituée par une pellicule sans tension.

2. Dispositif selon la revendication 1, **caractérisé en ce que** le dispositif de mesure implantable (8) est calibré avec un enveloppe finie (7).

3. Dispositif selon une des revendications 1 à 2, **caractérisé en ce que** la puce (1) comporte également un capteur de température (17).

4. Dispositif selon une des revendications 1 à 3, **caractérisé en ce que** la bobine inductive (3) est conformée en bobine plane déformable ou en bobine enroulée déformable.

5. Dispositif selon une des revendications 1 à 4, **caractérisé en ce que** la bobine inductive (3) est incluse sur ou dans un support souple.

6. Dispositif selon une des revendications 1 à 5, **caractérisé en ce que** les dimensions extérieures de la bobine inductive (3) sont inférieures à 18 mm, en particulier égales à environ 12 mm.

7. Dispositif selon une des revendications 1 à 6, **caractérisé en ce que** l'épaisseur du dispositif de mesure implantable est d'environ 2 mm ou moins.

8. Dispositif selon une des revendications 1 à 7, **caractérisé en ce que** la bobine inductive (3) est conformée de manière souple dans les trois dimensions.

9. Dispositif selon une des revendications 1 à 8, **caractérisé en ce qu'**une bobine inductive extracorporelle (9) apte à être connectée à un appareil d'interrogation/lecture (10) peut être fixée sur la tête du patient à proximité du dispositif de mesure implanté (8).

10. Dispositif selon une des revendications 1 à 9, **caractérisé en ce que** le dispositif télémétrique extracorporel est conformé en appareil portatif dans lequel est disposé l'appareil d'interrogation/lecture (10).

11. Dispositif selon la revendication 10, **caractérisé en ce que** l'appareil portatif comporte un dispositif indicateur (14).

12. Dispositif selon une des revendications 1 à 11, **caractérisé en ce que** le dispositif d'analyse (21) analyse les données mesurées à l'aide d'une courbe caractéristique associée au dispositif de mesure correspondant (8).

13. Dispositif selon une des revendications 1 à 12, **caractérisé en ce que** les données mesurées de plusieurs dispositifs de mesure implantables (8) peuvent être analysées par un dispositif d'analyse (21).

14. Dispositif selon la revendication 13, **caractérisé en ce que** les données mesurées comportent des identificateurs spécifiques qui sont associés aux dispositifs de mesure correspondants (8) et qui, lors de l'analyse, permettent d'appeler, à partir d'une mémoire de courbes caractéristiques (23), des courbes caractéristiques associées aux dispositifs de mesure correspondants (8).

15. Dispositif selon une des revendications 1 à 14, **caractérisé en ce que** les données mesurées contiennent la différence de pression entre la pression intracrânienne mesurée par le capteur de pression (5) et une pression pré-définie mesurée par un capteur de pression standard (16).

16. Dispositif selon une des revendications 1 à 15, **caractérisé en ce que** le dispositif d'analyse (21) est connecté à un capteur (24) pour détecter la pression de l'air ambiant.
